(19) **Europäisches Patentamt / European Patent Office / Office européen des brevets**

(11) **EP 3 718 553 B1**

(12) **EUROPEAN PATENT SPECIFICATION**

(45) Date of publication and mention
of the grant of the patent:
**11.09.2024 Bulletin 2024/37**

(21) Application number: **18882326.4**

(22) Date of filing: **27.11.2018**

(51) International Patent Classification (IPC):
*A61K 31/704* (2006.01)   *A61K 33/40* (2006.01)
*A61K 9/08* (2006.01)   *A61K 45/00* (2006.01)
*A61K 47/02* (2006.01)   *A61P 35/00* (2006.01)
*A61K 31/7068* (2006.01)   *A61K 33/00* (2006.01)
*A61K 33/04* (2006.01)   *A61K 33/06* (2006.01)
*A61K 33/243* (2019.01)   *A61K 33/26* (2006.01)
*A61K 33/30* (2006.01)   *A61K 45/06* (2006.01)

(52) Cooperative Patent Classification (CPC):
(C-Sets available)
A61K 31/704; A61K 9/0019; A61K 9/0053;
A61K 9/08; A61K 31/7068; A61K 33/243;
A61K 33/40; A61K 45/06; A61K 47/02; A61P 35/00

*(Cont.)*

(86) International application number:
**PCT/JP2018/043524**

(87) International publication number:
**WO 2019/107341 (06.06.2019 Gazette 2019/23)**

(54) **OZONE NANOBUBBLE ANTICANCER AGENT**

OZONNANOBLASEN-ANTIKREBSMITTEL

AGENT ANTICANCÉREUX DE NANOBULLES D'OZONE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.11.2017 JP 2017230554**

(43) Date of publication of application:
**07.10.2020 Bulletin 2020/41**

(73) Proprietor: **OPT Creation Inc.**
**Yokohama-shi, Kanagawa 230-0045 (JP)**

(72) Inventors:
• **IIDA Junichi**
**Yokohama**
**Kanagawa 230-0045 (JP)**
• **MINAGAWA Kyoji**
**Yokohama**
**Kanagawa 230-0045 (JP)**

(74) Representative: **Prüfer & Partner mbB**
**Patentanwälte · Rechtsanwälte**
**Sohnckestraße 12**
**81479 München (DE)**

(56) References cited:
**WO-A1-2016/021523   JP-A- 2009 084 258
JP-A- 2012 126 649**

• **SABINE A E HEIDER ET AL: "Carotenoid
biosynthesis and overproduction in
Corynebacterium glutamicum", BMC
MICROBIOLOGY, BIOMED CENTRAL LTD, GB,
vol. 12, no. 1, 10 September 2012 (2012-09-10),
pages 198, XP021140880, ISSN: 1471-2180, DOI:
10.1186/1471-2180-12-198**
• **DATABASE WPI Week 201781, Derwent World
Patents Index; AN 2017-80194N, XP055831748**

- **SIMONETTI, VINCENZO ET AL.: "Association of Ozone with 5-Fluorouracil and Cisplatin in Regulation of Human Colon Cancer Cell Viability:In Vitro Anti-Inflammatory Properties of Ozone in Colon Cancer Cells Exposed to Lipopolysaccharides", EVIDENCE-BASED COMPLEMENTARY AND ALTERNATIVE MEDICINE, vol. 2017, no. 7414083, 4 July 2017 (2017-07-04), pages 1 - 6, XP055615282**
- **SHIMASAKI TAKEO ET AL.: "Application of Molecular Medicine to Pancreatic Cancer", SUIZO, vol. 25, no. 1, 2010, pages 35 - 45, XP009520976, DOI: 10.2958/suizo.25.35**

(52) Cooperative Patent Classification (CPC): (Cont.)

C-Sets
**A61K 31/704, A61K 2300/00;
A61K 31/7068, A61K 2300/00;
A61K 33/243, A61K 2300/00;
A61K 33/40, A61K 2300/00**

**Description**

Technical Field

The present invention relates to ozone water comprising ozone nano-bubbles for use in treating or preventing a cancer, wherein the ozone water comprises Na ion, a Mg ion, a K ion and/or a Ca ion, wherein the ozone water further comprises at least one selected from the group consisting of a sulfur ion, a boron ion, a lithium ion, a silicon ion, a zinc ion, an iron ion and a strontium ion, wherein the ozone water is to be administered sequentially with other anticancer agent(s), and wherein said other anticancer agent(s) is cisplatin.

Background Art

Various anticancer agents have been developed and introduced into market; however, they are to kill cancer cells or suppress proliferation thereof and inevitably produce side effects on normal cells. In addition, the pharmacological effects of a new anticancer agent are investigated in combination with an existing anticancer agent in clinical trials, but it is difficult to develop the new anticancer agents. In the circumstance, molecular target drugs (primarily, antibody drug) targeting a specific gene product has been developed.

For example, an anti-CD20 antibody (generic name: rituximab) is used for treating CD20-positive non-Hodgkin's lymphoma and few side effects are produced on cells which express no CD20 on the surface thereof.

An anti-VEGF antibody (generic name: bevacizumab) inhibits the function of VEGF, thereby suppressing angiogenesis and proliferation/metastasis of tumors.

An anti-HER2 antibody (generic name: trastuzumab) exhibits an anti-tumor effect against breast cancer cells or the like highly expressing HER2 protein on the surface thereof.

Molecular target drugs are also used in a cancer immunotherapy to kill cancer cells through *in vivo* immune response. An anti-CTLA4 antibody (generic name: ipilimumab) and an anti-PD-1 antibody (generic name: nivolumab) cancel a function to suppress CTL

(cytotoxic T cell)'s attack against cancer cells, thereby indirectly serving as an anticancer agent.

These molecular target drugs (antibodies) are mainly constituted of naturally occurring amino acids, and it is not necessary to care about the effect on metabolites thereof; however, the excessive administration of them may , *in vivo,* induce an anti-idiotype antibody (an antibody recognizing an antibody administered as a drug as a foreign substance and binding thereto). In addition to this problem, because of a macromolecule, molecular target drugs cannot enter into the cells.

Patent Document 4 discloses an aqueous solution containing ozone nanobubbles.

Patent Document 5 refers to nanobubble water in connection with preventing or treating cancer.

Patent Document 6 refers to a composition containing ozone nanobubbles, which are formed by crushing ozone nanobubbles and which are present in a stabilized manner. Particularly, the composition is for suppressing bladder cancer growth.

Non Patent Document 4 refers to the association of different ozone concentration with 5-fluorouracil and cisplatin in human colon cancer cell in order to investigate possible anticancer synergistic effects.

Prior Art Documents

Patent Document

Patent Document 1: JP 2005-246293A
Patent Document 2: JP5261569B
Patent Document 3: WO2016/021523A
Patent Document 4: WO 2017/199827
Patent Document 5: JP 2009-84258
Patent Document 6: JP 2012-126649

Non Patent Document 1: in vivo 31: 579-583 (2017)
Non Patent Document 2: Journal of the Pharmaceutical Society of Japan 127 (11), 1837-1842, 2007-11-01
Non Patent Document 3: PNAS vol. 114, no. 5, E761-, 2017

Non Patent Document 4: Evidence-Based Complementary and Alternative Medicine vol. 2017, no. 7414083, pp. 1-6

Summary of Invention

The present invention is defined by the claims. Any subject-matter falling outside the scope of the claims is provided for information only.

Technical Problem

The present invention provides an anticancer agent, which is easily absorbed in cells and metabolized *in vivo* without changing a chemical structure and induces no immune response, and wherein the agent can be used in combination with other anticancer agent(s). Solution to Problem

As a result of intensive studies, the present inventors of the present application have found that ozone water storable for a long term has an anticancer effect; and furthermore, that the anticancer effect is significantly increased in combination with an existing anticancer agent.

Advantageous Effects of Invention

A pharmaceutical composition according to the present invention can be easily absorbed in cells and metabolized *in vivo* without changing a chemical structure, exert an anticancer effect without inducing an immune response, and be used in combination with other anticancer agent(s).

Brief Description of Drawings

[Fig. 1] Example 1, Tests on cell growth inhibition by NAnO3 for 10 types of cancer cell lines (2D culture)
[Fig. 2] Example 1, Tests on cell growth inhibition by NAnO3 for 10 types of cancer cell lines (3D culture)
[Fig. 3] Example 2, Examinations for the anti-tumor effect of a combination of NAnO3 and existing anticancer agents against MIAPaCa-2 pancreatic cancer cells in a concomitant administration
[Fig. 4] Example 2, Examinations for the anti-tumor effect of a combination of NAnO3 and existing anticancer agents against MIAPaCa-2 pancreatic cancer cells in a sequential administration
[Fig. 5] Example 3, Weight change of a test group
[Fig. 6] Example 3, Photograph of a group of test mice
[Fig. 7] Example 3, Tumor volume change of a test group

Description of Embodiments

The subject-matter of the present invention is set forth in the independent claim. Preferred embodiments are the subject-matter of the dependent claims. Hence, the present invention refers to the following subject-matter:
Ozone water comprising ozone nano-bubbles for use in treating or preventing a cancer,

wherein the ozone water comprises a Na ion, a Mg ion, a K ion and/or a Ca ion,
wherein the ozone water further comprises at least one selected from the group consisting of a sulfur ion, a boron ion, a lithium ion, a silicon ion, a zinc ion, an iron ion and a strontium ion, wherein the ozone water is to be administered sequentially with other anticancer agent(s), and wherein said other anticancer agent(s) is cisplatin.

The ozone water for use according to the invention, wherein a concentration of ozone in the ozone water is 1 ppm or more, preferably 10 ppm or more and more preferably 100 ppm or more.
The ozone water for use according to the invention, wherein the ozone water comprises bittern-containing water.
The ozone water for use according to the invention, wherein the cancer is selected from the group consisting of lung cancer, bladder cancer, prostate cancer, ovarian cancer, esophageal cancer, stomach cancer, cervical cancer and malignant lymphoma.
The ozone water for use according to the invention, wherein the ozone water is to be orally, intravenously or intratumorally administered.
The ozone water for use according to the invention, wherein the ozone water is to be administered once every day or three times per week.

The invention is defined by the claims.

The "ozone water" refers to water in which ozone ($O_3$) is dissolved. The ozone water for use according to the present invention is ozone nano-bubble water containing a high concentration of mineral, in which ozone nano-bubbles are maintained. The ozone water for use in the present invention comprises ozone nano-bubbles, further a Na ion, a Mg ion, a K ion and/or a Ca ion, and further at least one selected from the group consisting of a sulfur ion, a boron ion, a lithium ion, a silicon ion, a zinc ion, an iron ion and a strontium ion.

The "fine bubbles" refer to floating minute air bubbles with a particle size of 100 $\mu$m or less. The "ultrafine bubbles" refer to floating minute air bubbles with a particle size of less than 1 $\mu$m. The "nano-bubbles" being comprised in the ozone water for use according to the present invention may be "ultrafine bubbles". The "nano-bubbles" may contain floating minute air bubbles with a particle size of 1 nm or more and less than 1 $\mu$m, preferably 10 nm or more and less than 1 $\mu$m and more preferably several tens to several hundreds nm.

The floating minute air bubbles can be produced by, e.g. an ejector system, cavitation system, swirling flow system or pressure dissolution method.

The "bittern" refers to a mixture of minerals (powder or liquid) produced from seawater and containing magnesium chloride as a main component.

The "cancer" refers to a "malignant tumor" in a broad sense, including "carcinoma", "sarcoma" and hematological malignancy such as leukemia. The malignant tumor refers to a cell population which infiltrates into the peripheral tissue or develops metastasis among cell populations (tumor, benign tumor and malignant tumor) uncontrollably self-proliferated by genetic mutation to. The malignant tumors are pathologically classified into

1) Carcinoma: malignant tumor derived from the epithelium tissue
2) Sarcoma: malignant tumor derived from the non-epithelial tissue (e.g., bone, cartilage, fat, muscle, blood vessels)
3) Others: e.g., leukemia

The "cancer" as mentioned herein may include, but are not particularly limited to, carcinomas such as head and neck cancer (maxillary cancer, (upper, middle, lower) pharyngeal cancer, laryngeal cancer, tongue cancer, thyroid cancer), thoracic cancer (breast cancer, lung cancer (non-small cell lung cancer, small cell lung cancer)), digestive system cancer (esophageal cancer, stomach cancer, duodenal cancer, colorectal cancer (colon cancer, rectal cancer)), liver cancer (hepatocellular carcinoma, cholangiocellular carcinoma), gallbladder cancer, bile duct cancer, pancreatic cancer, anal cancer, urinary organ cancer (kidney cancer, ureteral cancer, bladder cancer, prostate cancer, penis cancer, testis (testicle) cancer), genital cancer (uterine cancer (cervical cancer, endometrial cancer), ovarian cancer, vulvar cancer, vaginal cancer) and skin cancer (basal cell carcinoma, squamous cell carcinoma).

Furthermore, the "cancer" as mentioned herein may include, but are not particularly limited to, fibrosarcoma, liposarcoma, myoma (e.g., leiomyosarcoma), hemangiosarcoma, Kaposi's sarcoma, lymphatic sarcoma, synovial sarcoma, osteosarcoma, extraosseous osteosarcoma and malignant peripheral nerve tumor.

Furthermore, the "cancer" as mentioned herein may include, but are not particularly limited to, hematological malignancies (for example, leukemia, malignant lymphoma, multiple myeloma).

In the present invention, the ozone water comprising ozone nano-bubbles, a Na ion, a Mg ion, a K ion and/or a Ca ion, and further at least one selected from the group consisting of a sulfur ion, a boron ion, a lithium ion, a silicon ion, a zinc ion, an iron ion and a strontium ion, is used in treating or preventing a cancer, and is to be administered sequentially with the other anticancer agent cisplatin.

The "anticancer agent" refers to a medicament for treating or preventing a cancer. The anticancer agents are classified, but are not particularly limited, into a molecular target drug, an alkylating agent, an antimetabolic drug, a plant alkaloid, an anticancer antibiotic, a platinum formulation, a hormonal agent, a biological response modifier and the like.

In the context of the present invention, the other anticancer agent is cisplatin.

The "molecular target drug" refers to a drug designed so as to target a specific gene product of cancer cells at a molecular level and efficiently acts on the targeted gene product. Examples of molecular target drugs (not claimed) include, but are not particularly limited to, ibritumomab tiuxetan, imatinib, everolimus, erlotinib, gefitinib, gemtuzumab ozogamicin, sunitinib, cetuximab, sorafenib, dasatinib, tamibarotene, trastuzumab, tretinoin, panitumumab, bevacizumab, bortezomib, lapatinib and rituximab. Further, ipilimumab and nivolumab fall within the molecular target drug, which cancel a function to suppress CTL (cytotoxic T cell)'s attack against cancer cells, thereby indirectly treating cancer with a cancer immunotherapy.

The "alkylating agent" functions to attach a mass of atomics named an alkyl group to the DNA of a cancer cell and allows the two helically twisted DNA strands to be abnormally joined to each other to prevent the DNAs from being coped. Examples of the alkylating agent include, but are not particularly limited to, ifosfamide, cyclophosphamide, dacarbazine, temozolomide, nimustine, busulfan, procarbazine, melphalan and ranimustine.

The "antimetabolic drug" refers to an anticancer agent, which is a substance analogous to a material for a nucleic acid in terms of chemical structure, suppresses synthesis of the DNA of cancer cells when they are divided/proliferated, thereby inhibiting metabolism of cancer cells and suppressing proliferation thereof. Examples of the antimetabolic drug include, but are not particularly limited to, enocitabine, capecitabine, carmofur, cladribine, gemcitabine, cytarabine, cytarabine ocfosfate, tegafur, tegafur uracil, tegafur gimeracil oteracil potassium, doxyfluridine, nelarabine, hydroxycarbamide, fluorouracil (5-FU), fludarabine, pemetrexed, pentostatin, mercaptopurin and methotrexate.

The "plant alkaloid" refers to an anticancer agent using a highly toxic plant component. Examples of the plant alkaloid include, but are not limited to, a microtubule inhibitor and a topoisomerase inhibitor (for example, irinotecan, etoposide, eribulin, sobuzoxane, docetaxel, nogitecan, paclitaxel, vinorelbine, vincristine, vindesine, vinblastine).

The "anticancer antibiotic" refers to an anti-tumor antibiotic produced from a mold or the like, contained in soil. Examples of the anticancer antibiotic include, but are not particularly limited to, actinomycin D, aclarubicin, amrubicin, idarubicin, epirubicin, zinostatin stimalamer, daunorubicin, doxorubicin, pirarubicin, bleomycin, peplomycin, mitomycin C, mitoxantrone and liposomal doxorubicin.

The "platinum formulation" refers to an anticancer agent containing platinum. Examples of the platinum formulation include, but are not particularly limited to, oxaliplatin, carboplatin, cisplatin and nedaplatin.

The "hormonal agent" refers to an anticancer agent suppressing secretion or function of *in vivo* hormone. Examples of the hormonal agent include, but are not particularly limited to, anastrozole, exemestane, estramustine, ethinyl estradiol, chlormadinone, goserelin, tamoxifen, dexamethasone, toremifene, bicalutamide, flutamide, prednisolone, fosfestrol, mitotane, methyltestosterone, medroxyprogesterone, mepitiostane, leuprorelin and letrozole.

The "biological response modifier" refers to an anticancer agent inducing an *in vivo* biological reaction for treatment. Examples of the biological response modifier include interferon-$\alpha$, interferon-$\beta$, interferon-y and interleukin.

Now, the present invention will be described in detail with reference to Examples; however, the present invention is not limited by these Examples.

Examples

<Method for producing ozone water>

Ozone water was prepared in accordance with a method described in International Publication No. WO2016/021523. More specifically, brine-containing water of a "deep-seawater bittern for professional use" (manufactured by Ako Kasei Co., Ltd.) was diluted three fold with water (tap water) and used in accordance with the description of Example 1. The "deep-seawater bittern for professional use" contains 12 to 30 wt% of Mg ions in terms of $MgCl_2$. Other than Mg ions, Ca ions (10 to 100 mg/liter) and Na ions (100 to 1000 mg/liter) are contained.

In the diluted aqueous mineral solution, ozone micro-bubbles with a particle size of 1.0 $\mu$m to 50 $\mu$m are generated. The micro-bubbles generated in the solution first looks milky white and then turn transparent as the micro-bubbles are crushed over time. In this manner, ozone water having a constant ozone concentration of 100 ppm or more was obtained (Non Patent Document 1).

The ozone water is available as NAnO3 from Opt Creation Inc.

<Example 1. Tests on cell growth inhibition by NAnO3 for 10 types of cancer cell lines>

The effect of stabilized ozone water NAnO3 on 10 types of cancer cell lines was investigated by 2D culture and 3D culture.

The following cell lines were obtained from ATCC (American Type Culture Collection) or JCRB cell bank and cultured in the mediums shown in Table 1.

[Table 1]

| Cell line | | Registration No. | Culture medium |
|---|---|---|---|
| Human lung adenocarcinoma cell line | A549 | ATCC CCL-185 | DMEM medium (GIBCO Cat# 11965092) |
| Human lung epithelial cell line | -11226 | ATCC CRL-5826 | RPMI1640 medium (GIBCO Cat# 11875119) |
| Human breast cancer cell line | MCF7 | ATCC HTB-22 | DMEM medium + insulin (0.01mg/ml) (SIGMA Cat# 10516-5ML) |
| Human breast cancer cell line | BT474 | ATCC HTB-20 | DMEM medium |
| Human pancreatic cancer cell lime | MIAPaCa-2 | ATCC CRL-1420 | DMEM medium |

(continued)

| Cell line | | Registration No. | Culture medium |
|---|---|---|---|
| Human colorectal cancer cell line | HT29 | ATCC HTB-38 | McCoy's 5A medium (GIBCO Cat# 11875119) |
| Human stomach cancer cell line | MKN-45 | JCRB0254 | RPMH640 medium |
| Human hepatoma cell line | HepG2 | ATCC HB-8065 | DMEM medium |
| Human prostate cancer cell line | DU-145 | ATCC HTB-81 | DMEM medium |
| Human ovary cancer cell line | SK-OV-3 | ATCC HTB-77 | McCoy's 5A medium |

All mediums contain a 10 v/v% PBS (Hana-nesco Bio) and 1 × Penicillin-Streptomycin-Glutamine (GIBCO Cat#10378016).

1.1 Evaluation by 2D culture

Each of the culture mediums (40 μL) was added to each well of Imaging Plate (96-Well, Tissue Culture Treated (BD FALCON Cat#353219). Thereafter, each of the cell suspensions (50 μL) was seeded (0.5 × 10$^4$ cells/well) and cultured in the presence of 5% $CO_2$ at 37°C.

1 day after the initiation of culture, each of the following test-substance solutions (10 μL) was added and the culturing was continued.

[Table 2]

| | |
|---|---|
| 1 | Ultra pure water, 100 v/v% |
| 2 | Sea-water mineral solution, 100 v/v% |
| 3 | NAnO3, 20 v/v% |
| 4 | NAnO3, 50 v/v% |
| 5 | NAnO3, 100 v/v% |

NAn03 was diluted with ultra pure water.
"Deep-sea water brine for professional use" described above was used as the a sea-water mineral solution (control).

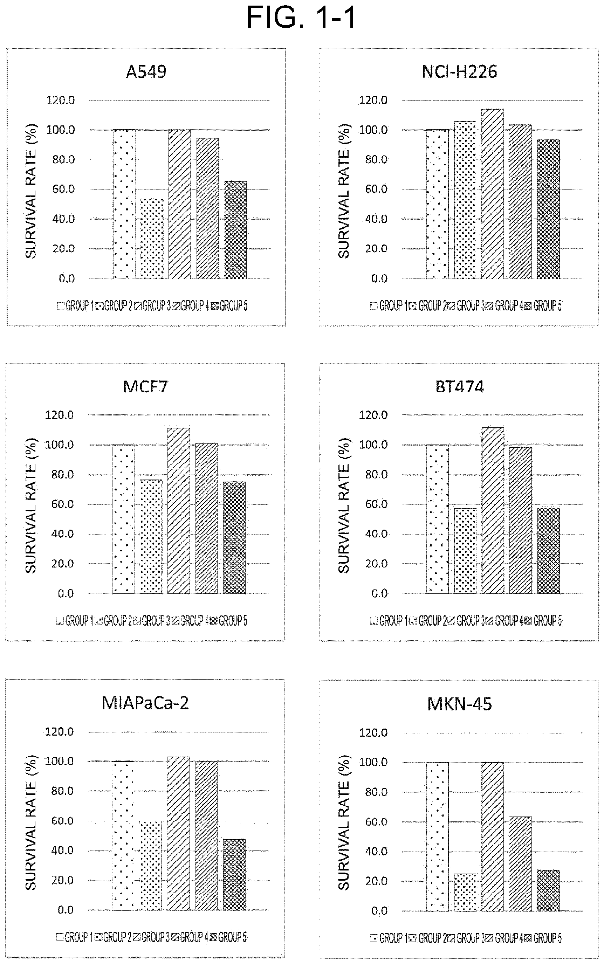

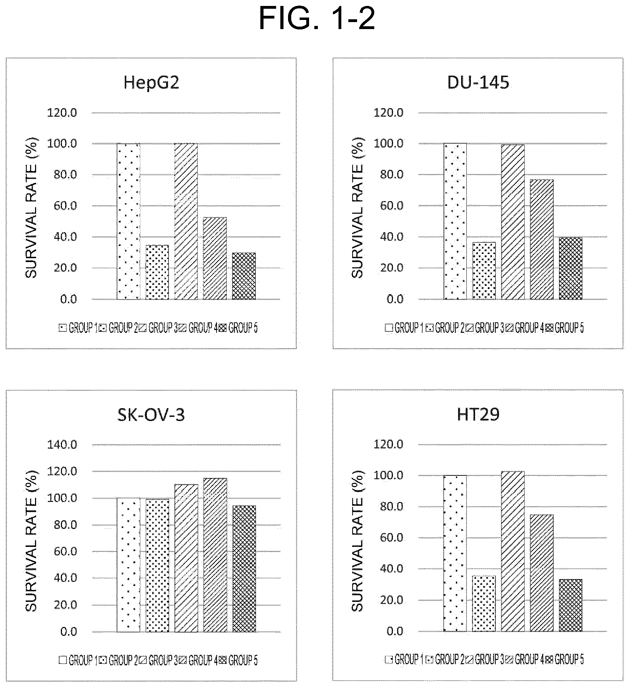

4 days after the initiation of culture, ATP concentration was determined with CellTiter-Glo (R) Luminescent Cell Viability Assay (CTG) (Promega Cat#G7572) and based on the ATP standard curve. Provided that the survival rate at the ATP concentration of the negative control group (test-substance solution 1) was regarded as 100%, the survival rates of each test group was calculated (Fig. 1).

1.2 Evaluation by 3D culture

Each of the culture mediums (40 μL) was added to each well of NanoCulture Plate (MH pattern, low adhesive: ORGANOGENIX). Thereafter, each of the cell suspensions (50 μL) was seeded at a density of 1 × 10$^4$ cells/well and cultured in the presence of 5% $CO_2$ at 37°C.

3 days after the initiation of culture, each of the test-substance solutions (10 μL) described in Table 2 was added and the culturing was continued.

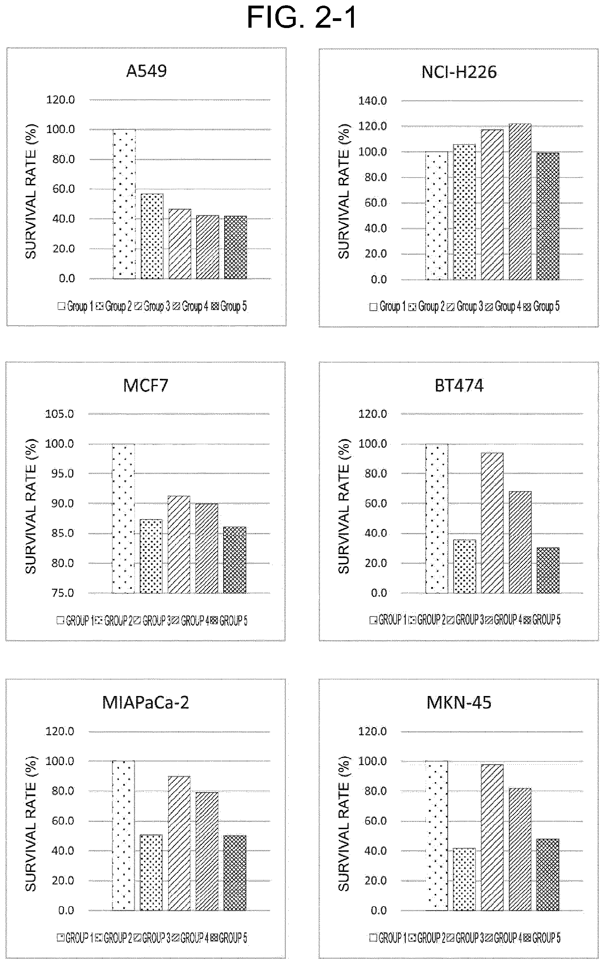

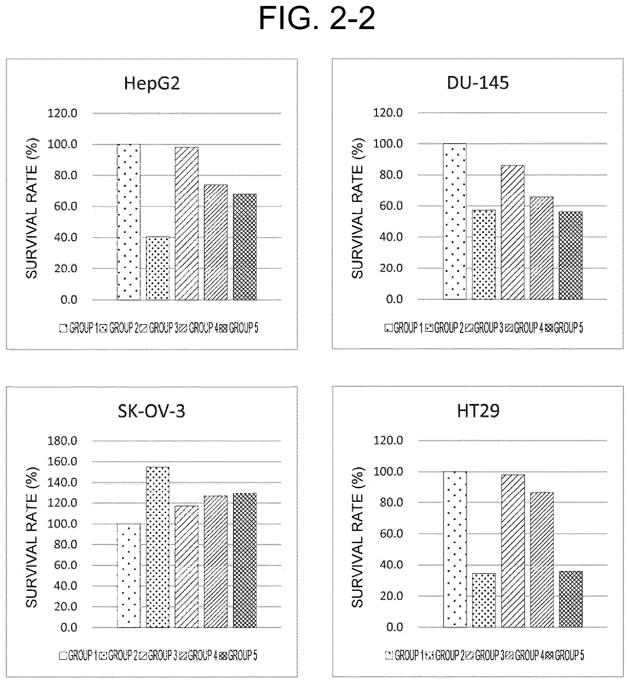

7 days after the initiation of culture, ATP concentration was determined with CellTiter-Glo (R) Luminescent Cell Viability Assay (CTG) (Promega Cat#G7572) and based on the ATP standard curve. Provided that the ATP concentration of the negative control group (test-substance solution 1) was regarded as a survival rate of 100%, the survival rates of each test group was calculated (Fig. 2).

As a result, both in 2D culture and 3D culture, the survival rates of a group (test-substance solution 5: NAnO3 concentration with a final of 10%) and a vehicle group (test-substance solution 2: sea-water mineral solution with a final concentration of 10%) decreased by 60% or less as compared with the negative control group (test-substance solution 1) in the cancer cell lines (including human lung adenocarcinoma cell line A549, human breast cancer cell line BT474, human pancreatic cancer cell line MIAPaCa-2, human stomach cancer cell line MKN-45, human hepatoma cell line HepG2, human prostate cancer cell line DU-145 and human colorectal cancer cell line HT29),. The survival rates of these cancer cells decreased in a dose-dependent manner. The effect of the vehicle group (test-substance solution 2, seawater mineral solution with a final concentration of 10%) to reduce survival rates of various cancer cells was considered to be equivalent to that of a group (test-substance solution 5: NAnO3 with a final concentration of 10%).

From the above results, it was observed that the growth suppression effect of NAnO3 on a cancer cell line varies in a dose-dependent manner under the test conditions; however, no significant difference was observed between test-substance solution 5 (10% NAnO3) and test-substance solution 2 (10% sea-water mineral solution).

<Example 2. Examinations for anti-tumor effects of a combination of NAnO3 and existing anticancer agents (doxorubicin, gemcitabine, cisplatin) against MIAPaCa-2>

The anti-tumor effect of a combination of NAnO3 and an existing anticancer agent (doxorubicin, gemcitabine, or cisplatin) against MIAPaCa-2 cells (see, Table 1) was examined. Only the combination with cisplatin is part of the invention. The examples concerning combinations with other anticancer agents are reference examples.

The following anticancer agents were used.

(1) Doxorubicin: doxorubicin hydrochloride for injection (adriacin for injection, 10 mg (Nippon Kayaku Co., Ltd.))
Doxorubicin intercalates between base pairs of DNA in tumor cells to inhibit DNA polymerase, RNA polymerase and topoisomerase II reaction. Doxorubicin exerts an anti-tumor effect by suppressing biosynthesis of both DNA and RNA in this manner. Doxorubicin is applied to malignant lymphoma, lung cancer, stomach cancer, gallbladder/bile duct cancer, pancreatic cancer, liver cancer, colorectal cancer, breast cancer, bladder cancer, endometrial cancer, osteosarcoma, multiple myeloma, various childhood cancers and the like.
(2) Gemcitabine: gemcitabine hydrochloride for injection (Gemzar for injection, 200 mg (Eli Lilly Japan))
Gemcitabine is taken by a DNA strand and another nucleoside is attached. In this manner, Gemcitabine stops extension of the DNA strand. As a result, apoptosis is induced to kill tumor cells. Gemcitabine is applied to non-small cell lung cancer, pancreatic cancer, biliary tract cancer, urothelial cancer and inoperable or recurrent breast cancer.
(3) Cisplatin: cisplatin formulation (Lander for injection 10 mg/20 mL (Nippon Kayaku Co., Ltd.))
Cisplatin binds to position N-7 of DNA constituent bases, guanine and adenine, to form a crosslink within a DNA chain, with the result that replication of DNA is prevented to kill cancer cells. Cisplatin is applied to various cancers such as lung cancer, bladder cancer, prostate cancer, ovarian cancer, esophageal cancer, stomach cancer, cervical cancer and malignant lymphoma. Cisplatin was mostly used in combination with other anticancer agent.

## 2.1 Experiment in concomitant administration

A cell suspension was prepared such that a MIAPaCa-2 viable cell count became $5 \times 10^4$ cells/mL. The cell suspension (1 mL) was seeded in each of wells of a 12 well plate, allowed to stand in a $CO_2$ incubator set at 37°C and 5% $CO_2$ and cultured for 18 hours or more to allow the cells to adhere to the bottom of the wells. After the completion of culture, the medium was removed from the wells and the following test-substance solutions (1 mL) was added. The same operation was also repeated after 48 hours and 72 hours.

[Table 3]

| | |
|---|---|
| 1 | DMEM medium |
| 2 | 10 v/v% Physiological saline / DMEM medium |
| 3 | 10 v/v% NAn03 / DMEM medium |
| 4 | 0. 1 $\mu$g/mL Doxorubicin / DMEM medium |
| 5 | 0. 04 $\mu$g/mL Gemcitabine / DMEM medium |
| 6 | 4 $\mu$g/mL Cisplatin / DMEM medium |
| 7 | 0. 1 $\mu$g/mL Doxorubicin + 10 v/v% NAn03 / DMEM medium |
| 8 | 0.04 $\mu$g/mL Gemcitabine + 10 v/v% NAn03 / DMEM medium |
| 9 | 4 $\mu$g/mL Cisplatin + 10 v/v% NAn03 /DMEM medium |

DMEM medium contains a 10 v/v% FBS (Hana-nesco Bio) and $1 \times$ Penicillin-Streptomycin-Glutamine (GIBCO Cat#10378016). The 10 v/v% physiological saline /DMEM medium was prepared by adding physiological saline (1 mL) to DMEM medium (9 mL). The 10 v/v% NAnO3/DMEM medium was prepared by adding NAnO3 (1 mL) to DMEM medium (9 mL). The anticancer agent/DMEM medium was prepared by diluting an anticancer agent 10 fold with physiological saline, and then adding DMEM medium (9 mL) to 1 mL of the solution. The anticancer agent + 10 v/v% NAnO3/DMEM medium was prepared by diluting an anticancer agent 10 fold with NAnO3, and thereafter, adding DMEM medium (9 mL) to the solution (1 mL).

After the completion of culture, the medium was removed and washing with PBS (-) was made. To each of the wells of the plate, a 0.005% neutral red (NR) (Kanto Chemical Co., Inc., Lot No.806W2294) D-MEM solution (1 mL) was added and the plate was allowed to stand in a $CO_2$ incubator for 3 hours. After 3 hours, the NR solution was removed and washing was made with PBS (-). To each well, 1 mL of a fixation/extraction liquid (ethanol 50% (v/v) glacial acetic acid 1% (v/v) aqueous solution) was added and shaking was made for 10 minutes. Thereafter, the value of the absorbance was measured by using a microplate reader (EL × 800, BIO-TEC INSTRUMENTS, INC., wavelength: 540 nm).

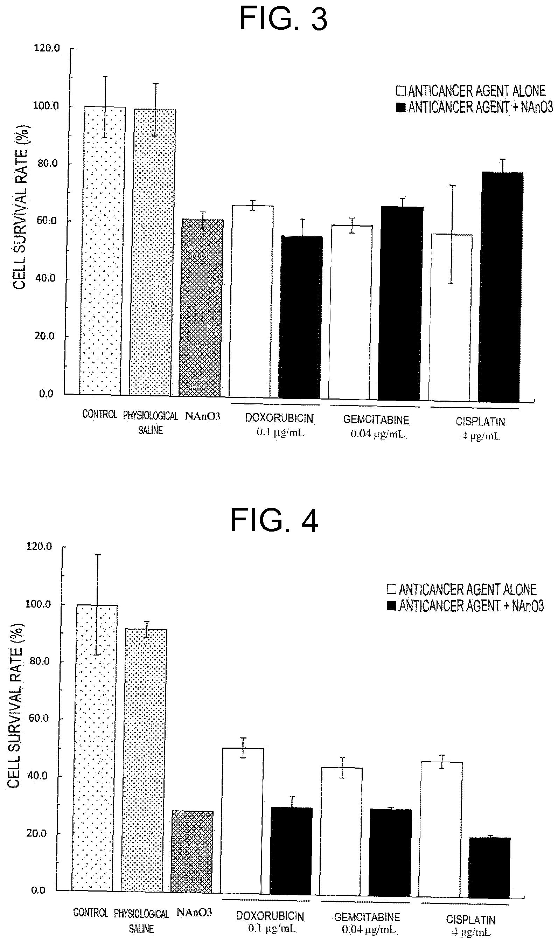

Provided that the absorbance of DMEM medium (test-substance solution 1) was regarded as 100%, the cell survival rate (%) of each test group was obtained (Fig. 3).

2.2 Experiment of sequential administration

A cell suspension was prepared such that MIAPaCa-2 viable cell count became $5 \times 10^4$ cells/mL. The cell suspension (1 mL) was seeded in each of wells of a 12 well plate, allowed to stand in a $CO_2$ incubator set at 37°C and 5% $CO_2$ and cultured for 18 hours or more to allow the cells to adhere to the bottom of the plate. After the completion of culture, the medium was removed from the plate and the following test-substance solutions were added.

[Table 4]

| | |
|---|---|
| 1 | DMEM medium 1mL |
| 2 | 10 v/v% Physiological saline / DMEM medium 1mL |
| 3 | 10 v/v% NAnO3 IDMEM medium 1mL |
| 4 | 0.1 μg/mL Doxorubicin / DMEM medium 1mL |
| 5 | 0.04 μg/mL Gemcitabine / DMEM medium 1mL |
| 6 | 4 μg/mL Cisplatin / DMEM medium 1mL |
| 7 | 0.1 μg/mL Doxorubicin / DMEM medium 0.9mL +NAn03 0.1mL |
| 8 | 0.04 μg/mL Gemcitabine / DMEM medium 0.9mL +NAn03 0.1mL |
| 9 | 4 μg/mL Cisplatin / DMEM medium 0.9mL +NAn03 0.1mL |

Test-substance solutions 1 to 6 were prepared in the same manner as in Section 2.1 and used. Test-substance solutions 7 to 9 were prepared by adding each anticancer agent/DMEM medium (0.9 mL) and, in several minutes later, adding 0.1 mL of NAnO3. The same operation was also repeated after 48 hours and 72 hours.

After the completion of culture, the medium was removed and washing with PBS (-) was made. To each of the wells of the plate, a 0.005% neutral red (NR) (Kanto Chemical Co., Inc., Lot No.806W2294) D-MEM solution (1 mL) was added and the plate was allowed to stand in a CO2 incubator for 3 hours. After 3 hours, the NR solution was removed and washing was made with PBS (-). To each plate, 1 mL of a fixation/extraction liquid (ethanol 50% (v/v), glacial acetic acid 1% (v/v) aqueous solution) was added and shaking was made for 10 minutes. Thereafter, the value of the absorbance was measured by using a microplate reader (EL × 800, BIO-TEC INSTRUMENTS, INC., wavelength: 540 nm).

Provided that the absorbance of DMEM medium (test-substance solution 1) was regarded as 100%, the cell survival rate (%) of each test group was obtained (Fig. 4).

As a result, in the experiment of concomitant administration, no difference was observed in the cell survival rate between the anticancer agents prepared with physiological saline and the anticancer agents prepared with NAnO3. Because of this, in the experiment of sequential administration, the exposure to the anticancer agents prepared with physiological saline was carried out, and then the exposure to the 10% anticancer agents prepared with NAnO3 were carried out to examine the cell survival rate. As a result, the cell survival rate of groups exposed only to doxorubicin, gemcitabine and cisplatin were 51.1%, 44.8% and 47.5%, respectively. In contrast, the cell survival rate of groups exposed also to NAnO3 were 30.3%, 30.3% and 21.3%, respectively. In this case, the apparent suppression was observed. Furthermore, cisplatin exhibited higher suppression than NAnO3 alone exhibiting a cell survival rate of 28.5%.

From the above results, in simultaneous exposure cases where cancer cells were exposed to each anticancer agent prepared with NAnO3, the proliferation of the cancer cells was not suppressed. In contrast, in the cases where the cancer cells were exposed to anticancer agents, and thereafter, exposed to NAnO3, the proliferation of the cells was apparently suppressed compared to the exposure to anticancer agents alone. In particular, in a combination use with cisplatin, it was presumed that a synergistic effect was produced.

<Example 3. Tests on tumor growth suppression by NAnO3 in MIAPaCa-2 (pancreatic cancer cells) transplanted mouse>

To examine the anti-tumor effect of NAnO3 *in vivo,* NAnO3 was administered to MIAPaCa-2 (pancreatic cancer cells) transplanted mice and its pharmacological effect was examined.

3.1 Experimental regimen

Animals (42 nude mice (BALB/c-nu (BALB/cAnN.Cg-foxn1$^{nu}$/CrlCrlj), female, 5 weeks old)) were purchased from Japan Charles River. At the day (Day 0 of the transplation) after the completion of the acclimatization period for a week, MIAPaCa-2 cells ($5.0 \times 10^6$ cells per mouse) were subcutaneously transplanted to the back of all of the animals in accordance with a routine method using Matrigel (Corning Matrigel basal membrane matrix). Day 6 after the completion of transplantation, individuals having a tumor volume of 150 mm$^3$ or more were selected and grouped into six (5 mice/group) so as to have the same average tumor volume. NAnO3 (1000 mg $\approx$ 1 ml) was forcibly administered via the oral route once a day for 14 days or intermittently (three times per week) administered intravenously and intratumorally. During the breeding period, the mice were raised in accordance with a routine method and the body weight and the tumor diameter were periodically measured. The experimental regimen is shown in Table 5.

[Table 5]

| | Group | Administration route | Individual No. | Dose/ liquid amount | Frequency of administration |
|---|---|---|---|---|---|
| 1 | Negative control oral administration group | Oral administration | 101-105 | 0mg/ 10ml/kg | Sequential administration for 14 days per month (once/day) |
| 2 | Low NAnO3 dosage oral administration group | Oral administration | 201-205 | 1000mg/ 10ml/kg | Sequential administration for 14 days per month (once/day) |
| 3 | Medium NAnO3 dosage oral administration group | Oral administration | 301-305 | 2000mg/ 10ml/kg | Sequential administration for 14 days per month (once/day) |
| 4 | High NAnO3 dosage oral administration group | Oral administration | 401-405 | 4000mg/ 10ml/kg | Sequential administration for 14 days per month (once/day) |
| 5 | NAnO3 intravenous administration group | Tail vein injection | 501-505 | 400mg/ 5ml/kg | Three times/week (once/day) |
| 6 | NAnO3 intratumoral administration group | Intratumoral injection | 601-605 | 400mg/ 2ml/kg | Three times/week (once/day) |

Sterile water for injection was given to the negative control oral administration group; NAn03 diluted with sterile water for injection was given to the NAn03 administration groups. Administration was carried out three times/week (Day 1, 3, 4, 8, 10 and 14) after the initiation of the experiment.

3.2 Measurement of body weight

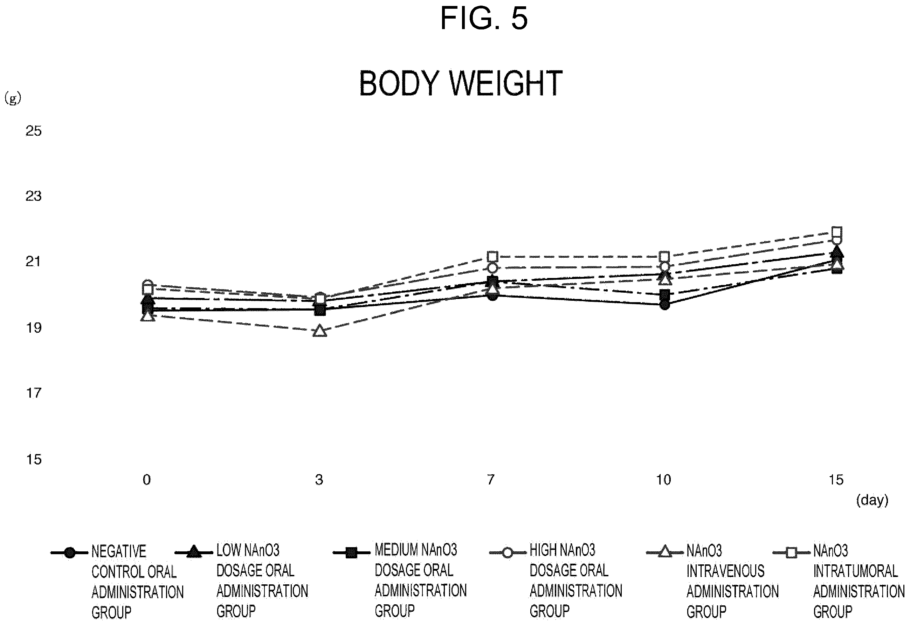

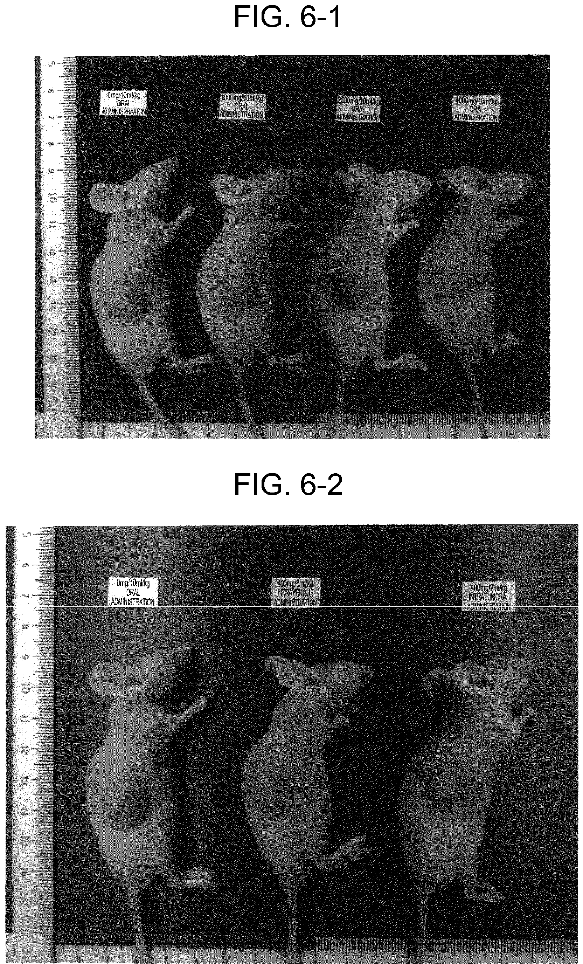

Body weights of individuals of each group were measured on Day 3, 7, 10 and 15 after the initiation of experiment (Table 6 and Fig. 5). As a result, the body weight changes shown in all test groups compared to Group 1 (control) in all the periods were almost the same. In any of the test groups, no abnormality was observed in general conditions. Photographs of mice on Day 15 belonging to each of test groups are shown in Fig. 6.

[Table 6]

Body weight measured (after grouping)                                                                                      Unit: g

| Name of group | Individual No. | Identification No. | 2016/12/12 day0 | 2016/12/15 day3 | 2016/12/19 day7 | 2016/12/22 day10 | 2016/12/27 day15 |
|---|---|---|---|---|---|---|---|
| Negative control oral administration group 1 | 101 | 23 | 19.18 | 19.18 | 19.78 | 19.79 | 20.75 |
| | 102 | 32 | 19.68 | 19.01 | 19.94 | 19.65 | 20.96 |
| | 103 | 19 | 18.99 | 19.12 | 19.59 | 18.98 | 20.67 |
| | 104 | 24 | 19.45 | 19.55 | 19.91 | 19.54 | 21.08 |
| | 105 | 33 | 20.26 | 20.69 | 20.63 | 20.33 | 21.58 |
| | Mean | | 19.51 | 19.51 | 19.97 | 19.66 | 21.01 |
| | ± S.D. | | 0.49 | 0.69 | 0.39 | 0.49 | 0.36 |
| Low NAnO3 dosage oral administration group 2 | 201 | 17 | 18.63 | 18.52 | 19.39 | 19.78 | 20.16 |
| | 202 | 37 | 20.73 | 20.66 | 21.21 | 21.26 | 21.65 |
| | 203 | 39 | 20.00 | 19.85 | 19.46 | 19.76 | 20.79 |
| | 204 | 1 | 19.81 | 19.67 | 20.49 | 20.86 | 21.31 |
| | 205 | 10 | 20.23 | 20.20 | 21.20 | 21.41 | 22.22 |
| | Mean | | 19.88 | 19.78 | 20.35 | 20.61 | 21.23 |
| | ± S.D. | | 0.78 | 0.80 | 0.89 | 0.80 | 0.79 |
| Medium NAnO3 dosage oral administration group 3 | 301 | 3 | 19.82 | 18.94 | 20.01 | 19.53 | 20.58 |
| | 302 | 40 | 20.24 | 19.62 | 20.54 | 20.23 | 20.80 |
| | 303 | 31 | 20.27 | 20.43 | 21.01 | 20.60 | 21.66 |
| | 304 | 9 | 19.48 | 19.79 | 20.44 | 20.00 | 21.10 |
| | 305 | 11 | 18.07 | 18.71 | 19.89 | 19.47 | 19.70 |
| | Mean | | 19.58 | 19.50 | 20.38 | 19.97 | 20.77 |
| | ± S.D. | | 0.90 | 0.69 | 0.45 | 0.48 | 0.72 |
| High NAnO3 dosage oral administration group 4 | 401 | 38 | 20.52 | 20.17 | 20.56 | 20.74 | 21.81 |
| | 402 | 5 | 20.95 | 20.01 | 21.25 | 21.07 | 22.40 |
| | 403 | 20 | 18.95 | 19.71 | 20.37 | 20.23 | 21.07 |
| | 404 | 8 | 21.40 | 20.48 | 21.66 | 22.07 | 22.59 |
| | 405 | 16 | 19.47 | 19.01 | 20.04 | 19.91 | 20.26 |
| | Mean | | 20.26 | 19.88 | 20.78 | 20.80 | 21.63 |
| | ± S.D. | | 1.02 | 0.56 | 0.66 | 0.84 | 0.97 |
| NAnO3 intravenous administration group 5 | 501 | 35 | 19.74 | 19.24 | 21.43 | 21.08 | 21.37 |
| | 502 | 26 | 18.79 | 18.68 | 18.77 | 20.32 | 21.01 |
| | 503 | 29 | 18.88 | 18.47 | 19.43 | 19.47 | 19.92 |
| | 504 | 18 | 19.99 | 19.28 | 21.29 | 20.99 | 21.64 |
| | 505 | 22 | 19.56 | 18.71 | 19.98 | 20.41 | 20.52 |
| | Mean | | 19.39 | 18.88 | 20.18 | 20.45 | 20.89 |
| | ± S.D. | | 0.53 | 0.36 | 1.16 | 0.65 | 0.69 |
| NAnO3 intratumoral administration group 6 | 601 | 4 | 20.27 | 20.20 | 20.98 | 21.05 | 21.72 |
| | 602 | 42 | 21.46 | 20.74 | 21.93 | 21.82 | 22.71 |
| | 603 | 28 | 19.77 | 19.50 | 21.09 | 20.67 | 21.57 |
| | 604 | 36 | 20.07 | 19.92 | 21.15 | 21.55 | 22.54 |
| | 605 | 13 | 19.22 | 18.96 | 20.42 | 20.30 | 20.78 |
| | Mean | | 20.16 | 19.86 | 21.11 | 21.08 | 21.86 |
| | ± S.D. | | 0.83 | 0.68 | 0.54 | 0.62 | 0.78 |

3.3 Measurement of tumor volume

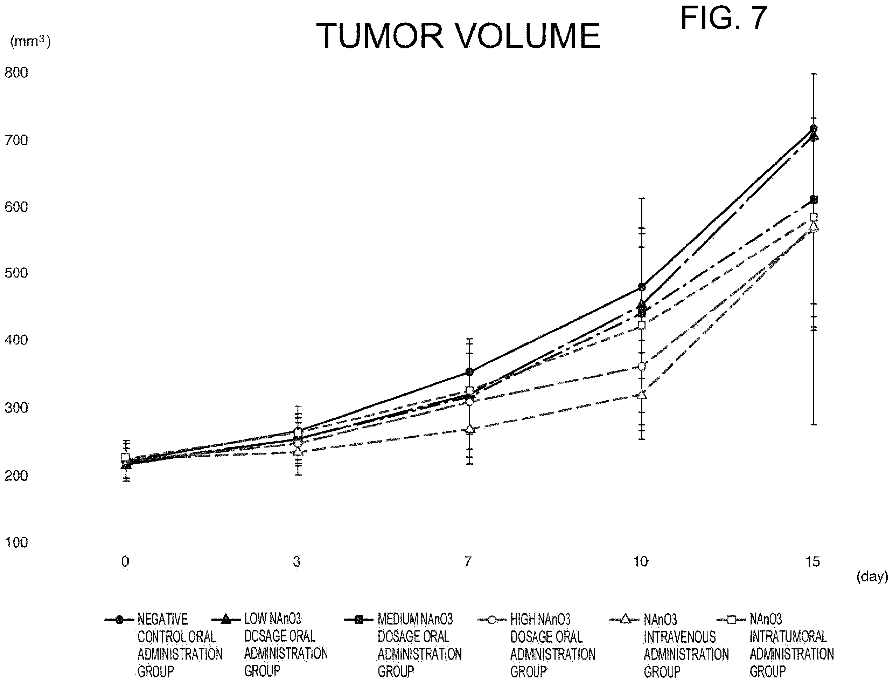

Transplantation sites were observed visually and by touch, and the major axis and minor axis of tumors were measured by an electronic caliper. Tumor volumes were calculated (table 7, Fig. 7) in accordance with the following expression:

$$[\text{major axis}] \, (\text{mm}) \times [\text{minor axis}] \, (\text{mm}) \times [\text{minor axis}] \, (\text{mm}) \times 0.5 = \text{tumor volume} \, (\text{mm}^3).$$

[Table 7]

Tumor volume measured (after grouping)

| Group | Individual No. | Identification No. | 2016/12/12 day0 Major axis (mm) | Minor axis (mm) | Tumor volume (mm³) | 2016/12/15 day3 Major axis (mm) | Minor axis (mm) | Tumor volume (mm³) | 2016/12/19 day7 Major axis (mm) | Minor axis (mm) | Tumor volume (mm³) | 2016/12/22 day10 Major axis (mm) | Minor axis (mm) | Tumor volume (mm³) | 2016/12/27 day15 Major axis (mm) | Minor axis (mm) | Tumor volume (mm³) |
|---|---|---|---|---|---|---|---|---|---|---|---|---|---|---|---|---|---|
| Negative control oral administration group 1 | 101 | 23 | 7.39 | 7.06 | 184.17 | 7.71 | 7.33 | 207.12 | 9.13 | 8.93 | 364.04 | 10.06 | 9.99 | 501.99 | 12.61 | 11.09 | 775.44 |
| | 102 | 32 | 7.96 | 7.22 | 207.47 | 8.48 | 8.36 | 296.33 | 9.18 | 9.13 | 382.61 | 9.94 | 9.81 | 478.29 | 11.21 | 10.41 | 607.40 |
| | 103 | 19 | 7.96 | 7.35 | 215.01 | 8.35 | 8.06 | 271.22 | 9.24 | 8.98 | 372.56 | 11.17 | 10.36 | 599.44 | 13.08 | 11.92 | 929.25 |
| | 104 | 24 | 7.89 | 7.77 | 238.17 | 8.03 | 7.96 | 254.40 | 8.75 | 8.62 | 325.08 | 9.61 | 8.97 | 386.61 | 11.00 | 10.69 | 628.52 |
| | 105 | 33 | 9.68 | 7.10 | 243.98 | 9.93 | 7.79 | 301.30 | 10.04 | 8.10 | 329.36 | 11.76 | 8.65 | 439.96 | 13.28 | 9.80 | 637.71 |
| Mean | | | — | — | 217.76 | — | — | 266.07 | — | — | 354.73 | — | — | 481.26 | — | — | 715.66 |
| ± S.D. | | | — | — | 24.22 | — | — | 38.06 | — | — | 26.00 | — | — | 79.17 | — | — | 136.55 |
| Low NAnO3 dosage oral administration group 2 | 201 | 17 | 7.45 | 7.15 | 190.43 | 7.55 | 7.15 | 192.99 | 8.45 | 7.95 | 267.03 | 9.68 | 9.54 | 440.50 | 11.97 | 11.22 | 753.44 |
| | 202 | 37 | 8.62 | 6.92 | 206.39 | 9.32 | 7.68 | 274.86 | 10.41 | 8.88 | 410.44 | 13.39 | 9.87 | 652.21 | 16.23 | 10.85 | 955.32 |
| | 203 | 39 | 8.30 | 7.23 | 216.93 | 8.30 | 7.80 | 252.49 | 8.30 | 7.14 | 211.57 | 8.30 | 7.14 | 211.57 | 9.35 | 8.19 | 313.58 |
| | 204 | 1 | 8.33 | 7.53 | 236.16 | 8.51 | 8.11 | 279.86 | 8.99 | 8.90 | 356.05 | 10.02 | 9.89 | 490.04 | 12.44 | 11.90 | 880.81 |
| | 205 | 10 | 9.18 | 7.31 | 245.27 | 9.98 | 7.52 | 282.19 | 10.15 | 8.50 | 366.67 | 10.87 | 9.37 | 477.18 | 12.93 | 9.95 | 640.05 |
| Mean | | | | | 219.04 | — | — | 256.48 | — | — | 322.35 | — | — | 454.30 | — | — | 708.64 |
| ± S.D. | | | | | 22.16 | — | — | 37.39 | — | — | 80.92 | — | — | 158.18 | — | — | 251.63 |
| Medium NAnO3 dosage oral administration group 3 | 301 | 3 | 7.37 | 7.27 | 194.76 | 7.48 | 7.34 | 201.49 | 7.81 | 7.54 | 222.01 | 8.45 | 8.20 | 284.09 | 8.82 | 8.57 | 323.89 |
| | 302 | 40 | 7.93 | 7.20 | 205.55 | 8.35 | 7.81 | 254.66 | 9.74 | 8.28 | 333.88 | 9.93 | 9.39 | 437.77 | 10.63 | 10.05 | 536.83 |
| | 303 | 31 | 7.81 | 7.50 | 219.66 | 8.22 | 7.97 | 261.07 | 8.82 | 8.25 | 300.16 | 10.09 | 9.49 | 454.35 | 11.98 | 10.48 | 657.88 |
| | 304 | 9 | 8.21 | 7.56 | 234.62 | 8.22 | 7.69 | 243.05 | 8.86 | 7.91 | 277.18 | 10.21 | 9.94 | 504.39 | 11.98 | 11.50 | 792.18 |
| | 305 | 11 | 9.33 | 7.45 | 258.92 | 9.84 | 7.81 | 300.10 | 11.10 | 9.06 | 455.56 | 12.56 | 9.25 | 537.33 | 14.21 | 10.22 | 742.11 |
| Mean | | | | | 222.70 | — | — | 252.07 | — | — | 317.76 | — | — | 443.59 | — | — | 610.58 |
| ± S.D. | | | | | 25.19 | — | — | 35.48 | — | — | 87.14 | — | — | 97.56 | — | — | 187.18 |
| High NAnO3 dosage oral administration group 4 | 401 | 38 | 7.81 | 7.12 | 197.96 | 8.17 | 7.44 | 226.12 | 8.88 | 8.00 | 284.16 | 9.50 | 8.15 | 315.51 | 11.65 | 10.47 | 638.54 |
| | 402 | 5 | 8.34 | 7.02 | 205.50 | 8.83 | 7.33 | 237.21 | 8.83 | 7.81 | 269.30 | 8.86 | 7.89 | 275.78 | 10.15 | 8.58 | 373.60 |
| | 403 | 20 | 8.87 | 7.05 | 220.43 | 8.96 | 7.59 | 258.08 | 9.79 | 7.88 | 303.95 | 10.50 | 8.03 | 338.52 | 13.02 | 9.62 | 602.46 |
| | 404 | 8 | 8.05 | 7.61 | 233.10 | 8.24 | 7.61 | 238.60 | 8.46 | 7.92 | 265.33 | 9.03 | 8.97 | 363.28 | 10.19 | 10.03 | 512.56 |
| | 405 | 16 | 8.68 | 7.77 | 262.02 | 8.89 | 7.99 | 283.77 | 10.84 | 8.97 | 436.10 | 11.17 | 9.66 | 521.17 | 12.98 | 10.48 | 712.80 |
| Mean | | | — | — | 223.80 | — | — | 248.76 | — | — | 311.77 | — | — | 362.85 | — | — | 567.99 |
| ± S.D. | | | — | — | 25.30 | — | — | 22.70 | — | — | 71.14 | — | — | 94.18 | — | — | 130.38 |
| NAnO3 intravenous administration group 5 | 501 | 35 | 7.78 | 7.15 | 198.87 | 7.89 | 7.15 | 201.68 | 8.50 | 7.23 | 222.16 | 9.33 | 7.37 | 253.39 | 10.98 | 8.59 | 405.10 |
| | 502 | 26 | 7.71 | 7.29 | 204.87 | 7.93 | 7.44 | 219.48 | 8.41 | 8.19 | 282.06 | 9.32 | 8.87 | 366.63 | 11.29 | 10.78 | 656.00 |
| | 503 | 29 | 7.85 | 7.50 | 220.78 | 7.96 | 7.68 | 234.75 | 8.22 | 7.84 | 252.62 | 8.54 | 8.19 | 286.41 | 9.64 | 9.61 | 445.14 |
| | 504 | 18 | 7.78 | 7.73 | 232.44 | 7.78 | 7.73 | 232.44 | 8.03 | 7.74 | 240.53 | 8.51 | 8.20 | 286.11 | 10.57 | 10.45 | 577.14 |
| | 505 | 22 | 9.67 | 7.46 | 269.08 | 10.25 | 7.52 | 289.82 | 11.05 | 7.96 | 350.07 | 12.47 | 8.07 | 406.05 | 15.36 | 10.15 | 791.21 |
| Mean | | | — | — | 225.21 | — | — | 235.63 | — | — | 269.49 | — | — | 319.72 | — | — | 574.92 |
| ± S.D. | | | — | — | 27.86 | — | — | 33.02 | — | — | 50.04 | — | — | 63.82 | — | — | 157.35 |
| NAnO3 intratumoral administration group 6 | 601 | 4 | 8.32 | 6.97 | 202.10 | 8.44 | 7.68 | 248.91 | 8.76 | 7.76 | 263.75 | 9.83 | 8.27 | 336.15 | 12.66 | 9.84 | 612.91 |
| | 602 | 42 | 7.88 | 7.20 | 204.25 | 8.97 | 7.47 | 250.27 | 9.79 | 7.69 | 289.47 | 10.03 | 7.76 | 301.99 | 11.59 | 8.43 | 411.82 |
| | 603 | 28 | 7.95 | 7.47 | 221.81 | 8.31 | 7.99 | 265.26 | 9.32 | 8.85 | 364.98 | 10.25 | 10.17 | 530.07 | 11.92 | 11.75 | 822.85 |
| | 604 | 36 | 9.51 | 6.92 | 227.70 | 10.16 | 7.37 | 275.93 | 10.91 | 8.86 | 428.22 | 12.91 | 9.84 | 625.01 | 16.21 | 10.59 | 908.96 |
| | 605 | 13 | 8.22 | 8.11 | 270.32 | 8.62 | 8.11 | 283.48 | 8.72 | 8.32 | 301.81 | 9.15 | 8.38 | 321.28 | 7.33 | 6.60 | 159.65 |
| Mean | | | — | — | 225.24 | — | — | 264.77 | — | — | 329.65 | — | — | 422.90 | — | — | 583.24 |
| ± S.D. | | | — | — | 27.50 | — | — | 15.30 | — | — | 66.51 | — | — | 145.61 | — | — | 305.45 |

Average tumor volume of Group 1 (control) was 217.76 ± 24.22 mm³ at the time of grouping and increased up to 715.66 ± 136.55 mm³ at the end of the administration period. From this, it was determined that MIAPaCa-2 pancreatic cancer cells transplanted were successfully proliferated. The average tumor volume of oral administration group 2 (a dose of 1000 mg/kg) were 219.04 ± 22.16 mm³ (grouping time) and 708.64 ± 251.63 mm³ (the end of the administration period); that of oral administration group 3 (a dose 2000 mg/kg), were 222.70 ± 25.19 mm³ (grouping time) and 610.58 ± 187.18 mm³ (the end of the administration period); and furthermore, that of oral administration group 4 (a dose of 4000 mg/kg) were 223.80 ± 25.30 mm³ (grouping time) and 567.99 ± 130.38 mm³ (the end of the administration period). No significant difference was observed; however, an increase of tumor volume tends to be suppressed in a dose-dependent manner. In intravenous administration group 5, the average tumor volume was 225.21 $\pm$ 27.86 mm$^3$ (grouping time) and 574.92 $\pm$ 157.35 mm$^3$ (the end of the administration period). A tendency of significant growth suppression was shown. In intratumoral group 6, the average tumor volume was 225.24 $\pm$ 27.50 mm$^3$ (grouping time) and 583.24 $\pm$ 305.45 mm$^3$ (the end of the administration period). Tendency of growth suppression was shown compared to Group 1 (control). Particularly, a significant reduction of tumor volume was confirmed in a single case at the time of measurement on Day 15.

From the above results, NAnO3 exhibits a cancer cell growth suppression effect by any of oral administration, intravenous administration, and intratumoral administration, and significant side effects such as weight loss were not observed.

<Example 4. Tests on intratumoral DNA damage in MIAPaCa-2 pancreatic cancer cells-transplanted mice with NAnO3>

Based on the results of Example 3, 6 nude mice (female, 5 weeks old (BALB/c-nu)), to which MIAPaCa-2 human pancreatic cancer cells were transplanted, were intratumorally administered with NAnO3 (400 $\mu$L/body) three times per week. 14 days after the completion of administration, the tumor tissues were excised out and subjected to histopathological examination. The rate of cells positive to gamma-H2AX was calculated.

The tumor tissues were fixed with 10% neutral formalin and embedded in paraffin in accordance with a routine method, and subjected to immunohistochemical staining using an anti-gamma-H2AX antibody (Histone H2AX phosphorylated antibody (Ser 139), OxiSelect (R) DNA double strand cleavage staining kit, company: Cell Biolab). The obtained specimens were observed through objective lens (40 times magnification) and 3 sites per specimen were randomly selected and photographed. Thereafter, the number of cells where double stranded DNA was cleaved was counted (Table 8).

[Table 8]

Gamma-H2AX positive cells

| Sample ID | 4 | | | 5 | | | 6 | | |
|---|---|---|---|---|---|---|---|---|---|
| | 1 | 2 | 3 | 1 | 2 | 3 | 1 | 2 | 3 |
| Number of positive cells | 4 | 4 | 4 | 3 | 4 | 5 | 1 | 2 | 1 |
| Total cell number | 286 | 349 | 347 | 297 | 335 | 341 | 334 | 268 | 392 |
| Positive cell ratio (%) | 1.399 | 1.146 | 1.153 | 1010 | 1.194 | 1.466 | 0.299 | 0.746 | 0.255 |
| Average positive cell ratio (%) | 1.233 | | | 1.233 | | | 0.433 | | |
| Standard deviation | 0.144 | | | 0.229 | | | 0.272 | | |
| Maximum value | 1.399 | | | 1.466 | | | 0.746 | | |
| Minimum value | 1.146 | | | 1.010 | | | 0.255 | | |

Gamma-H2AX (phosphorylated histone protein) is a marker for double stranded DNA cleavage. From the above results, intratumoral double stranded DNA cleavage was confirmed even if it is extremely slight. It is so far known that reactive oxygen species such as ozone strongly damages DNA. Various anticancer agents such as cisplatin and doxorubicin are deemed to work based on the mechanism of damaging DNA. However, these drugs have a problem of giving damage to normal cells. In contrast, NAnO3 was found to exert an anticancer effect without virtually damaging DNA.

Industrial Applicability

The ozone water for use according to the invention of the present application is easily absorbed in cells and metabolized *in vivo* without changing a chemical structure; and exerts an anticancer effect without inducing an immune response and can be used in combination with other anticancer agent(s). If it is desired to enhance the efficacy including DNA damage of the composition, the concentrations of minerals and ozone can be changed or light, ultrasonic wave and the like can be used in combination (Non Patent Document 2). It has been currently found that Inducer, reactive oxygen (ROS: Reactive Oxygen Species), is important for activating drugs including nivolumab (Non Patent Document 3).

NAnO3 that can serve as mild reactive oxygen is also suitable for use in combination with these drugs. Further, in order to enhance the efficacy of the drugs, light, ultrasonic wave and the like can be used in combination. Alternatively, e.g., a radiation therapy and a cancer immunotherapy may be used in combination.

**Claims**

1. Ozone water comprising ozone nano-bubbles for use in treating or preventing a cancer,

   wherein the ozone water comprises a Na ion, a Mg ion, a K ion and/or a Ca ion,
   wherein the ozone water further comprises at least one selected from the group consisting of a sulfur ion, a boron ion, a lithium ion, a silicon ion, a zinc ion, an iron ion and a strontium ion,
   wherein the ozone water is to be administered sequentially with other anticancer agent(s), and
   wherein said other anticancer agent(s) is cisplatin.

2. The ozone water for use according to claim 1, wherein a concentration of ozone in the ozone water is 1 ppm or more, preferably 10 ppm or more and more preferably 100 ppm or more.

3. The ozone water for use according to claim 1, wherein the ozone water comprises bittern-containing water.

4. The ozone water for use according to any one of claims 1 to 3, wherein the cancer is selected from the group consisting of lung cancer, bladder cancer, prostate cancer, ovarian cancer, esophageal cancer, stomach cancer, cervical cancer and malignant lymphoma.

5. The ozone water for use according to any one of claims 1 to 4, wherein the ozone water is to be orally, intravenously or intratumorally administered.

6. The ozone water for use according to any one of claims 1 to 4, wherein the ozone water is to be administered once every day or three times per week.


**Patentansprüche**

1. Ozonwasser umfassend Ozon-Nanoblasen zur Verwendung bei der Behandlung oder Vorbeugung eines Krebses,

   wobei das Ozonwasser ein Na-Ion, ein Mg-Ion, ein K-Ion und/oder ein Ca-Ion umfasst,
   wobei das Ozonwasser ferner mindestens eines ausgewählt aus der Gruppe bestehend aus einem Schwefel-Ion, einem Bor-Ion, einem Lithium-Ion, einem Silizium-Ion, einem Zink-Ion, einem Eisen-Ion und einem Strontium-Ion, umfasst,
   wobei das Ozonwasser nacheinander mit anderen (anderem) Antikrebsmittel(n) verabreicht werden soll, und
   wobei das andere (die anderen) Anti-Krebs-Mittel(n) Cisplatin ist (sind).

2. Ozonwasser zur Verwendung nach Anspruch 1, wobei die Ozonkonzentration im Ozonwasser 1 ppm oder mehr, vorzugsweise 10 ppm oder mehr und noch bevorzugter 100 ppm oder mehr beträgt.

3. Ozonwasser zur Verwendung nach Anspruch 1, wobei das Ozonwasser bitterstoffhaltiges Wasser umfasst.

4. Ozonwasser zur Verwendung nach einem der Ansprüche 1 bis 3, wobei der Krebs ausgewählt ist aus der Gruppe bestehend aus Lungenkrebs, Blasenkrebs, Prostatakrebs, Eierstockkrebs, Speiseröhrenkrebs, Magenkrebs, Gebärmutterhalskrebs und malignem Lymphom.

5. Ozonwasser zur Verwendung nach einem der Ansprüche 1 bis 4, wobei das Ozonwasser oral, intravenös oder intratumoral verabreicht werden soll.

6. Ozonwasser zur Verwendung nach einem der Ansprüche 1 bis 4, wobei das Ozonwasser einmal täglich oder dreimal wöchentlich verabreicht werden soll.


**Revendications**

1. Eau d'ozone comprenant des nanobulles d'ozone pour une utilisation dans le traitement ou la prévention d'un cancer,

   dans laquelle l'eau d'ozone comprend un ion Na, un ion Mg, un ion K et/ou un ion Ca,

dans laquelle l'eau d'ozone comprend en outre au moins un élément choisi dans le groupe constitué par un ion soufre, un ion bore, un ion lithium, un ion silicium, un ion zinc, un ion fer et un ion strontium,

dans laquelle l'eau d'ozone est destinée à être administrée séquentiellement avec un ou des autre(s) agent(s) anticancéreux, et

dans laquelle ledit autre(s) agent(s) anticancéreux est le cisplatine.

2. L'eau d'ozone destinée à être utilisée selon la revendication 1, dans laquelle une concentration d'ozone dans l'eau d'ozone est de 1 ppm ou plus, de préférence de 10 ppm ou plus et plus préférablement de 100 ppm ou plus.

3. L'eau d'ozone destinée à être utilisée selon la revendication 1, dans laquelle l'eau d'ozone comprend de l'eau contenant de matière amère.

4. L'eau d'ozone destinée à être utilisée selon l'une quelconque des revendications 1 à 3, dans laquelle le cancer est choisi dans le groupe constitué par un cancer du poumon, un cancer de la vessie, un cancer de la prostate, un cancer de l'ovaire, un cancer de l'oesophage, un cancer de l'estomac, un cancer du col de l'utérus et un lymphome malin.

5. L'eau d'ozone destinée à être utilisée selon l'une quelconque des revendications 1 à 4, dans laquelle l'eau d'ozone est destinée à être administrée par voie orale, intraveineuse ou intratumorale.

6. L'eau d'ozone destinée à être utilisée selon l'une quelconque des revendications 1 à 4, dans laquelle l'eau d'ozone est destinée à être administrée une fois par jour ou trois fois par semaine.

# FIG. 1-1

# FIG. 1-2

# FIG. 2-1

A549

NCI-H226

MCF7

BT474

MIAPaCa-2

MKN-45

# FIG. 2-2

# FIG. 3

# FIG. 4

FIG. 5

BODY WEIGHT

Legend:
- NEGATIVE CONTROL ORAL ADMINISTRATION GROUP
- LOW NAnO3 DOSAGE ORAL ADMINISTRATION GROUP
- MEDIUM NAnO3 DOSAGE ORAL ADMINISTRATION GROUP
- HIGH NAnO3 DOSAGE ORAL ADMINISTRATION GROUP
- NAnO3 INTRAVENOUS ADMINISTRATION GROUP
- NAnO3 INTRATUMORAL ADMINISTRATION GROUP

EP 3 718 553 B1

# FIG. 6-1

# FIG. 6-2

FIG. 7

TUMOR VOLUME

(mm³)

800

700

600

500

400

300

200

100

0          3          7          10          15

(day)

NEGATIVE CONTROL ORAL ADMINISTRATION GROUP / LOW NAnO3 DOSAGE ORAL ADMINISTRATION GROUP / MEDIUM NAnO3 DOSAGE ORAL ADMINISTRATION GROUP / HIGH NAnO3 DOSAGE ORAL ADMINISTRATION GROUP / NAnO3 INTRAVENOUS ADMINISTRATION GROUP / NAnO3 INTRATUMORAL ADMINISTRATION GROUP

EP 3 718 553 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005246293 A
- JP 5261569 B
- WO 2016021523 A
- WO 2017199827 A
- JP 2009084258 A
- JP 2012126649 A

**Non-patent literature cited in the description**

- *in vivo,* 2017, vol. 31, 579-583
- *Journal of the Pharmaceutical Society of Japan,* 01 November 2007, vol. 127 (11), 1837-1842
- *PNAS,* 2017, vol. 114 (5), E761
- *EVIDENCE-BASED COMPLEMENTARY AND ALTERNATIVE MEDICINE,* vol. 2017 (7414083), 1-6